Europäisches Patentamt

European Patent Office

Office européen des brevets

(19)

(11) Publication number: **0 105 195**
**A2**

# (12) EUROPEAN PATENT APPLICATION

(21) Application number: **83108569.1**

(22) Date of filing: **31.08.83**

(51) Int. Cl.³: **A 61 L 2/06,** A 23 L  3/00,
A 23 B  9/00

(30) Priority: **01.09.82  US 413652**

(43) Date of publication of application: **11.04.84**
**Bulletin 84/15**

(84) Designated Contracting States: **BE DE FR GB IT**

(71) Applicant: **G.D. Searle & Co., Box 1045, Skokie,**
**Illinois 60076 (US)**

(72) Inventor: **Young, James George, 2729 Oak Avenue,**
**Northbrook Illinois (US)**
Inventor: **Colliopoulos, John Andrew, 1114 Dobson,**
**Evanston Illinois (US)**
Inventor: **Robin, Martin Steven, 806 Redbud Lane,**
**Wilmette Illinois 60091 (US)**
Inventor: **Paul, David Barner, 42 Terrace Lane, Lake**
**Zurich Illinois (US)**

(74) Representative: **Wolff, Hans Joachim, Dr.jur. Dipl.-Chem.**
**et al, Bell, Wolff & Bell Rechtsanwälte**
**Postfach 80 01 40 Adelonstrasse 58, D-6230 Frankfurt am**
**Main 80 (DE)**

(54) **Process for sanitizing psyllium hydrophilic mucilloid by extrusion.**

(57) The present invention relates to a method for sanitizing
psyllium hydrophilic mucilloid by exposure to moist heat under
elevated pressure in an extruder.

EP 0 105 195 A2

Our No. 24 128


G. D. Searle & Co.
Skokie, Ill. / USA


PROCESS FOR SANITIZING

PSYLLIUM HYDROPHILIC MUCILLOID BY EXTRUSION


BACKGROUND OF THE INVENTION


a) Field of the Invention

The present invention relates to a method for sanitizing psyllium hydrophilic mucilloid. In particular, it relates to a method consisting of subjecting psyllium hydrophilic mucilloid to moist heat under elevated pressure while extruding to ambient temperature and pressure.

The term psyllium seed is used in the generic sense to refer to seeds of the _Plantagi_ genus and this term is recognized (Roy L. Whistler and James N. BeMiller, _Industrial Gums_, Academic Press, New York and London (1959)p. 438) to include seeds of species such as _Plantago_ _psyllium_, _Plantago_ _ovata_, _Plantago_ _indica_, _Plantago_ _lanseolato_, and _Plantago_ _major_ as representative but not exclusive examples.

Psyllium hydrophilic mucilloid·is the husk obtained from the psyllium seed and may be milled or unmilled. Psyllium hydrophilic mucilloid contains natural mucillage and forms a gelatinous mass on contact with water.  It is useful in the treatment of constipation by acting as a fecal softner and also as a demulcent in the presence of inflamed mucosa.

Generally, psyllium hydrophilic mucilloid is separated from the seed by the application of pressure to the whole seed; the husk is separated, sanitized, and then incorporated into laxative products with or without further processing.

Untreated psyllium hydrophilic mucilloid has an average microbial count of about 50,000 counts/gram. In addition, naturally occurring microbial pathogens and insect eggs are occasionally present. In order to make psyllium hydrophilic mucilloid suitable for oral consumption, these contaminants must be greatly reduced or eliminated (sanitization).

b) Prior Art

Psyllium hydrophilic mucilloid is sanitized by a number of methods. Treatment with ethylene oxide (ETO) reduces the microbial count, but results in undesirable residues such as ethylene chlorohydrin and ethylene glycol. In addition, ETO, a known mutagen, is generally not allowed in foods nor recommended for use by pregnant women. (See Fed. Reg. Vol. 43 No. 122, Fri, June 23, 1978) Lastly, ETO-treated psyllium husk must be stored from 2 to 6 months to dissipate chemical residues. Likewise, chlorination is used but produces undesirable taste and alters swelling characteristics of the psyllium hydrophilic mucilloid. U.S. Patent 3,992,147 describes a method for sterilizing psyllium hydrophilic mucilloid using aqueous isopropanol with hydrogen peroxide. However, this method is expensive and not readily adaptable to large scale commercial production. While steam sterilization is known in the art, the tendency for psyllium hydrophilic mucilloid to swell on contact with water has rendered this method unworkable.  The U.S.P. defines steam sterilization as heating in an autoclave employing saturated steam under pressure for at least 15 minutes at a minimum temperature of 121°C.

SUMMARY OF THE INVENTION

It is therefore an object of this invention to develop a method for sanitization of psyllium hydrophilic mucilloid which is readily adaptable to commercial use, is free from harmful residues and undesirable taste, reduces the need for mandatory storage, yet retains the appropriate physical characteristics necessary for efficacy.

Current commercially available psyllium hydrophilic mucilloid is either untreated or ETO sanitized.  Untreated psyllium hydrophilic mucilloid contains an average microbial count of about 50,000 counts/gram and also may contain microbial pathogens, and insect eggs.  For consumption purposes it is highly desirable to substantially reduce the microbial count and eliminate pathogens and insect eggs, especially if the product is to be marketed worldwide where ex-U.S. requirements may be more restrictive.

ETO does reduce the microbial count to around 5,000 to 20,000 counts/gram but the treated psyllium hydrophilic mucilloid must be stored from 2 to 6 months to dissipate chemical residues such as ethylene chlorohydrin and ethylene glycol. These residues have been implicated as possible carcinogens and it would be desirable to eliminate them altogether. The ambient storage process, however, only reduces residues to around 100ppm. That level may be unacceptable for some uses, for example, in Sweden, not more than 5ppm of ethylene chlorohydrin is allowed in a given psyllium hydrophilic mucilloid sample. In addition, Sweden requires not more than 100 microbial counts/gram for psyllium hydrophilic mucilloid. Obviously, a method for reducing the microbial count, pathogens and insect eggs as well as eliminating ETO and its residues would be highly desirable.

In accordance with these objects, it has been discovered that psyllium hydrophilic mucilloid that is extruded using moist heat such that the internal product temperature is from about 80°C to about 120°C at a pressure greater than about 500 psi, that is extruded to ambient temperature and pressure meets these requirements. The psyllium hydrophilic mucilloid is not significantly changed in physical characteristics and becomes more homogeneous within the parameters of this invention.

Extrusion may be defined as shaping by forcing through a specially designed die opening. Extrusion therefore is primarily oriented toward the continuous forming of plastic, soft or food products through a die. Several designs are possible for extruders. The simplest is a ram or piston extruder. Other forms of extruders are flighted screw(s) or worm(s) rotating within a sleeve or barrel. The action of the flight on the screw pushes the material to be extruded forward and creates pressure behind a die so that it extrudes through the opening. Heat is applied directly by such means as steam injection or indirectly through jackets and by dissipation of the mechanical energy due to the shearing occurring within the extruded material.

For a discussion of the history of food extruders see for example Extrusion of Food, CRC Press, Judson M. Harper pp 3-6.

A food extruder consists of a screw(s) which rotates in a tightly fitting cylindrical barrel. Raw ingredients may be preground and blended before being placed in the feed end of the extrusion screw. These ingredients may be partially heated and moisturized in a preconditioning chamber which can be of the atmospheric or pressure cooker type. The action of the flights on the screw pushes the food product forward and in so doing, works and mixes the constituents into a viscous dough-like mass. Heat is added to the food dough as it passes through the screw by one or more of three mechanisms: (1) viscous dissipation of mechanical energy being added to the shaft of the screw, (2) heat transfer from steam or electrical heaters surrounding the barrel, and (3) direct injection of steam which is mixed with the dough in the screw.

As the moist, hot material moves through the extruder, the pressure within the barrel increases due to a restriction at the discharge of the barrel. The restriction is caused by one or more orifices or shaped openings called a die. Discharge pressures typically vary between 400 to 2200 psi. At these elevated pressures, boiling or flashing of moisture does not occur within the confines of the barrel because the pressure exceeds the vapor pressure of water at the extrusion temperature. Once the mass emerges from the die, the pressure is released causing the product to expand with extensive flashing of moisture. The loss of moisture and drop in pressure results in cooling of the product, solidification and often retention of its expanded shape.

While moist heat is generally accepted as a sterilization method, it is also recognized that moist heat is unacceptable for psyllium hydrophilic mucilloid because of the tendency of psyllium hydrophilic mucilloid to swell prematurely. Indeed, outside the parameters of this invention that is precisely what happens.

The invention is accomplished by placing psyllium hydrophilic mucilloid into the feed hopper of a standard food extruder such as the Creusot-Loire double screw or the Wenger single screw type. The barrel jacket is operated at a temperature so as to result in an internal product temperature of from about 80°C to about 120°C and a pressure greater than about 500 psi and the extruder operated in a normal manner. Pressure is a direct function of the screw speed, viscosity, temperature of the product, feed rate and the percent open area of the die used in the extrusion process. One skilled in the art may readily vary these functionalities to achieve a pressure in excess of about 500 psi. While the instant invention will work at any pressure above about 500 psi, the upper limits of pressure will most likely be proportional to the temperature, and the strength of the particular extruder used, it being recognized that these functionalities will set practical upper limits on the process which will vary from circumstance to circumstance. In general the practical upper limits will be around 2,200 psi. Likewise, the practical lower limits of pressure may vary slightly from 500 psi from machine to machine within limits understood in the art. Below about 80°C, and about 500 psi., as expected, the psyllium hydrophilic mucilloid becomes rubber-like and impossible to mill further. Extrusion above about 120°C yields a product with swell volume and viscosity below acceptable levels. A preferred internal temperature is between 100-120°C. The product may then be milled and sized ready for use.

The product of the instant invention compares favorably with products sanitized in conventional manner such that bulk density, viscosity, swell volume, particle

9

size distribution, and color remain relatively comparable within acceptable limits. The milled product does show an improvement in that it becomes more homogeneous in terms of overall appearance when compared to previous products. One skilled in the art could readily distinguish, on microscopic examination, a sample of prior art product versus the extruded product.

The following examples further illustrate details for the method of sanitizing of psyllium hydrophilic mucilloid. The invention is not to be construed as being limited either in spirit or in scope by these examples. Those skilled in the art will readily understand that known variations of the conditions and processes of the following are possible.

Brief Description of the Drawings

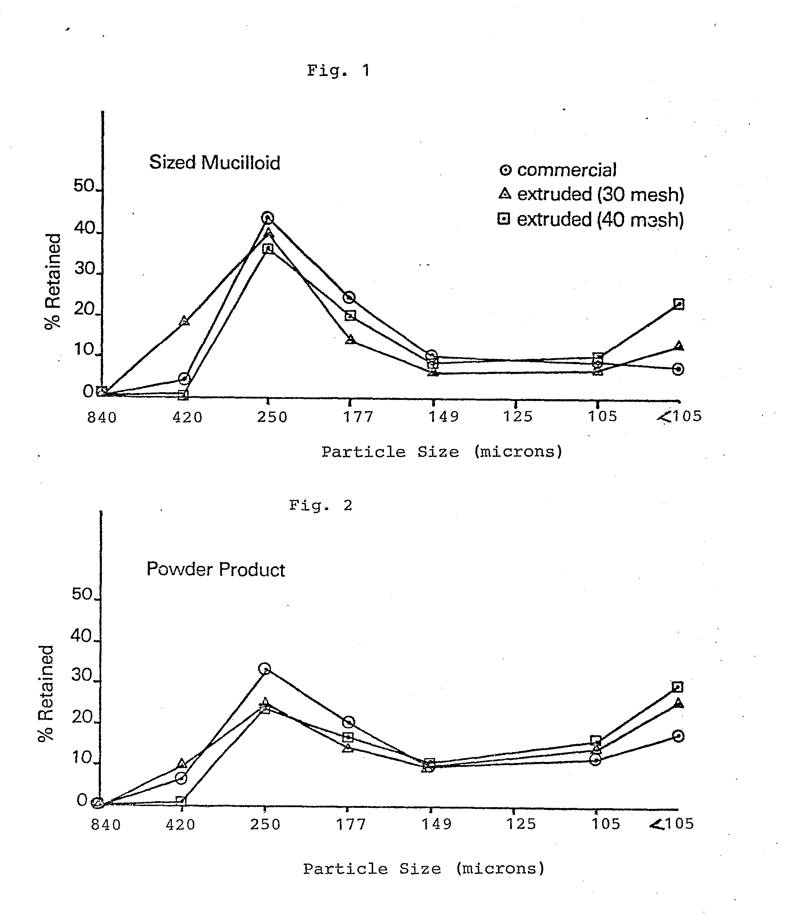

Referring to the drawings in example 6: Figure 1 is a chart comparing particle size distribution of the extruded and milled psyllium hydrophilic mucilloid versus the commercial psyllium hydrophilic mucilloid. Fig. 2 is a chart comparing particle size distribution for commercial product containing powdered psyllium hydrophilic mucilloid versus product containing the extruded and milled psyllium hydrophilic mucilloid.

DESCRIPTIONS OF THE PREFERRED EMBODIMENTS

Example 1             Microbial Count Reduction in Extruded

                    Psyllium Hydrophilic Mucilloid Using a Creusot-Loire BC 45 double screw extruder, commercial untreated psyllium hydrophilic mucilloid having a microbial count of 51,500/gram was extruded at different temperatures.

| Temperature | Total Microbial Counts/Gram |
|---|---|
| Untreated | 51,500 |
| 60° C** | ** |
| 80° C* | 95 |
| 109° C* | 8 |
| 120° C* | Ca. 1 |
| 137° C* | Ca. 1 |

*In extruded products all pathogens were destroyed.

**Below about 80° C the material turned into rubber-like mass that was not possible to mill or determine microbial count.

The milled extruded product, when compared to commercial ETO-treated and milled product, is more homogeneous and has comparable bulk density and flow characteristics. On microscopic examination, at approximately 40X, the commercial sized product appears as flat flakes with characteristic "fingerprint" patterns on the lateral surfaces, while the extruded product appears as chunks, with no pattern on the surface.

Example 2    Effect of temperature on Swell Volume of

extruded psyllium hydrophilic mucilloid.

The minimum acceptable swell volume is about 30 mls. per

0.5 g.

| Temperature | Swell Volume(ml/0.5 g.) |
| --- | --- |
| Untreated | 30-35 |
| 80° C | 32 |
| 109° C | 32 |
| 120° C | 32-33.5 |
| 125° C | 27 |
| 137° C | 25-26 |
| 157° C | 22.5 |
| 165° C | 17-18 |

Example 3    Effect of temperature on viscosity of

extruded psyllium hydrophilic mucilloid.

Average 24 hour viscosity of current commercial mucilloid at ambient temperature is about 1100 CPS.  One rounded teaspoonful is added to 8 oz. of water, stirred and allowed to stand at room temperature for 24 hours.

| Temperature | Viscosity (CPS) |
|---|---|
| 80°C | 1487.5-1605.5 |
| 109°C | 1635.0 |
| 118°C | 1210.0 |
| 125°C | 810.0 |
| 127°C | 722.5 |
| 137°C | 555.0 |
| 157°C | 610.0 |
| 165°C | 610.0 |

Example 4    Effect of temperature on moisture content

of extruded psyllium hydrophilic mucilloid.

Current processes do not appreciably affect the water content of psyllium hydrophilic mucilloid.  While not critical to the invention, water content will affect the ultimate weight of the product which is sold by weight.

| Temperature | % Water |
|---|---|
| Untreated | 9-12.0 |
| 80° C | 16.0 |
| 109° C | 11.8 |
| 120° C | 10.9 |
| 137° C | 11.4-12.5 |
| 162° C | 9.4 |
| 165° C | 10.0 |
| 177° C | 7.7- 7.9 |
| 186° C | 8.1 |

Example 5     Microbial Properties of Extruded (100° C)

Psyllium Hydrophilic Mucilloid

| | | Total Aerobic Microorganism Count | Inoculated Bacterial Pathogens | |
|---|---|---|---|---|
| | | | E. coli | Salmonella |
| Lot # 1 | Unextruded | 118,600/G | $4.14 \times 10^6/_g$ | $3.56 \times 10^6/_g$ |
| | Extruded | 100/g | negative | negative |
| Lot # 2 | Unextruded | 51,600/g | $4.16 \times 10^6/_g$ | $3.88 \times 10^6/_g$ |
| | Extruded | 100/g | negative | negative |
| Lot # 3 | Unextruded | 26,200/g | $4.13 \times 10^6/_g$ | $3.43 \times 10^6/_g$ |
| | Extruded | 100/g | negative | negative |

Example 6    The particle size distribution of extruded psyllium hydrophilic mucilloid vs. commercial product is shown in figures 1 and 2 of the drawings.

Claims:

1.   A process for sanitizing psyllium hydrophilic mucilloid without substantially affecting the useful properties, comprising subjecting psyllium hydrophilic mucilloid to moist heat such that the internal product temperature is from about 80°C to about 120°C, under conditions such that the pressure is above about 500 psi while concurrently extruding to ambient temperature and pressure.

2.   A process according to Claim 1 where the internal product temperature is 100°C to 120°C.

3.   A psyllium hydrophilic mucilloid composition prepared according to Claim 1.

0105195

1/1

Fig. 1

Sized Mucilloid

⊙ commercial
△ extruded (30 mesh)
⊡ extruded (40 mesh)

% Retained

Particle Size (microns)

Fig. 2

Powder Product

% Retained

Particle Size (microns)